# EUROPEAN PATENT SPECIFICATION

(11) **EP 0 941 041 B1**
(45) Date of publication and mention of the grant of the patent: **09.01.2002**
(21) Application number: 97910029.4
(22) Date of filing: 08.10.1997
(51) Int. Cl.: A61F 13/58, A61F 13/62

(54) **LAMINATE TAPE**
MEHRSCHICHTIGES BAND
BANDE LAMINEE

(30) Priority: 22.11.1996 DE 19648463
(43) Date of publication of application: 15.09.1999
(73) Proprietor: MINNESOTA MINING AND MANUFACTURING COMPANY, St. Paul, Minnesota 55133-3427 (US)
(72) Inventor: VON JAKUSCH, Egbert, D-42653 Solingen (DE)
(74) Representative: Wilhelm, Stefan
(86) International application number: US9718206
(87) International publication number: WO9822069

(56) References cited:
- EP-A- 0 276 970
- EP-A- 0 321 232
- EP-A- 0 648 483
- WO-A-97/25891
- US-A- 5 053 028

## Description

### Background and Field of the Invention

The present invention relates to a laminate tape which can be used in disposable absorptive articles such as diapers for adults and children and which is particularly suited as a closure tape for disposable diapers but is also useful as a closure means for other articles to be held in a folded condition. The laminate tape forming the subject matter of the instant invention comprises a mechanical fastening member cooperating with a complementary mechanical fastening member of a two-part mechanical fastening device. The laminate tape is fastened to one end of the article or object to be held in a folded condition by the laminate tape, and the mechanical fastening member complementary to the mechanical fastening member of the laminate tape is attached to the other end of the article or object to be held in a folded condition.

In closure systems of disposable diapers, it is known to realize the cohesion of the diaper by use of mechanical fastening devices. Diapers provided with such fastening devices are known from U.S. Pat. Nos. 4,869,724 and 5,053,028 as well as EP 0 321 232 B1, EP 0 324 578 A1, EP 0 374 730 A2 and EP 0 563 457 A1. All of these known closure systems are structured in the configuration of a Y comprising three flaps connected in a Y-shape. Two of said three flaps have their mutually confronting inner sides provided with adhesive faces by which the Y-shaped closure is fixed by adhesion in the region of its edge on both sides of the diaper. The third flap, projecting from the edge in the closed condition of the diaper, on one side carries one member of a two-part mechanical fastening device and is in hooking engagement with the complementary other mechanical fastening member. In the non-used or respective closed condition of the diaper, said (closure) flap comprising said one mechanical fastening member is laid on one of the flaps fixedly connected to the diaper. In this condition, the closure flap is releasably held to the respective other flap by means of a pressure-sensitive adhesive or the like adhesive. This adhesive connection exists outside that region of the closure flap where said one mechanical fastening member is arranged.

Besides the above outlined Y-shaped closure systems, the state of the art also includes adhesive closure tapes folded in a Z-shaped configuration. These adhesive closure tapes comprise a laminate strip consisting of a support layer and an adhesive layer arranged on one of the two sides of the support layer. When viewed in its lengthwise direction, the laminate strip is divided into three portions, and in the Z-folded condition, said three portions are arranged above each other. One of the two outer portions of the laminate strip is fixedly connected to the outer layer of the diaper by the adhesive layer of the support layer. Thus, in the Z-folded condition of the adhesive closure tape, the adhesive faces of the two other portions are facing each other. To allow for easy release of the Z-folded adhesive tape, a releasing layer is arranged between the mutually confronting adhesive faces. This layer is provided as a film of which one side is fixedly connected to the adhesive layer of one of said two portions of the closure strip, and of which the other side has release properties and in the Z-folded condition is arranged opposite to the adhesive layer of the last portion of the closure strip. Due to the requirement to provide one of the three portions of this known closure strip with a film having a releasing upper side and being connected to the adhesive layer, this known type of Z-folded closure adhesive tapes is relatively expensive in manufacture.

From EP 0 648 483 A1, there is known a Z-folded closure strip, provided e.g. for disposable diapers, which in the longitudinal direction of the strip comprises successive portions separated from each other by kinking or folding lines. The first portion can be connected to one end of the diaper by an adhesive layer. Within the second portion following the first portion, the closure tape is substantially free from adhesive. On the inner side of the third portion, which in the Z-folded condition of the closure strip is arranged opposite to the second portion, a mechanical fastening member is provided. The cohesion of the Z-shaped structure of this known closure strip is obtained by small adhesive zones which serve for releasable mutual connection of respectively confronting side faces of the three portions. In this configuration, the adhesive zone by which the third portion comprising the mechanical fastening member is releasably held on the second portion of the closure strip, is arranged outside the mechanical fastening member. The manufacture of this known closure strip is not without problems since it is required to use a support strip which is only partially provided with adhesive. In this regard, it is more convenient and thus less expensive for the manufacture and the provision of the manufacturing materials to start with a support material having an adhesive layer arranged over the full surface area of one side. Further, the mechanical fastening member of this known closure strip foldable into a Z-shape is formed as an integral component of the support strip. Also this feature renders the manufacturing process more complicated and increases the costs for the manufacturing materials. It is an object of the invention to provide a laminate strip, adapted to be folded into a Z-shaped configuration and useful as a closure system particularly for liquid-absorbing articles such as diapers for babies or adults, which is provided with one of the two mechanical fastening members of a two-part mechanical fastening device and can be manufactured in a simple and thus inexpensive manner.

### Disclosure of the Invention

The instant invention comprises a laminate strip having a lengthwise direction and comprising a support layer and an adhesive layer on a first face of the support layer, the laminate strip comprising a first, a second and a third portion which in this order are arranged successively to each other when viewed in said lengthwise direction, and a first mechanical fastening member of a two-part mechanical fastening device, said two-part mechanical fastening device comprising two mechanical fastening members which can be connected to each other while generating a retaining force, wherein
- the first mechanical fastening member comprises a lower side and an upper side, said upper side being capable of cooperating with the second mechanical fastening member of the two-part mechanical fastening device,
- the first mechanical fastening member has its lower side fixedly connected to the adhesive layer within the second or third portions of the laminate strip, and
- the laminate strip is folded into a zigzag shape in such a manner that said second and said third portions of the laminate strip have their adhesive layers arranged opposite each other on the first face of the support layer and that the first mechanical fastening member is arranged between said second and third portions of the laminate strip, wherein the upper side of the first mechanical fastening member is releasably attached to the adhesive layer of that portion of the laminate strip which is arranged opposite to the portion of the laminate strip fixedly connected to the lower side of the first mechanical fastening member.

The laminate tape of the invention comprises a laminate strip provided with a support layer and an adhesive layer arranged thereon. Particularly, the adhesive layer extends over the complete width and length of the support layer of the laminate strip, i.e. the adhesive layer is continuous. This laminate strip can be folded into a zigzag- or Z-shaped structure, thus generating three portions which follow each other in the lengthwise direction of the laminate strip and have kinking or bending lines arranged therebetween transversely to said longitudinal direction. Preferably, these three portions have substantially the same dimension in the longitudinal direction of the laminate strip. As a result of the Z- or S-shaped folding structure of the laminate strip, the adhesive layer in one of the two lateral end portions is arranged on the outer side of the folded laminate strip. In this portion, the folded laminate strip can be fastened to one end of the article (diaper) provided for the absorption of, e.g., liquid and being held together by the laminate strip. To facilitate the further explanation of the invention, it will be assumed that this end portion is the first portion of the laminate strip.

In the second or third portions of the laminate strip of the laminate tape of the invention, there is arranged a first mechanical fastening member of a two-part mechanical fastening device. Said first mechanical fastening member has a lower side by which the mechanical fastening member is fixedly connected to the adhesive layer of the second or third portion. The upper side facing towards the lower side of the first mechanical fastening member is of a structured configuration so as to cooperate with the upper side of the second mechanical fastening member which is formed complementary to the first mechanical fastening member. The upper side of the first mechanical fastening member comprises pins which are provided, e.g., with hooks or enlarged heads, while the upper side of the second mechanical fastening member is provided, e.g. with loops so that, when the upper sides of the two mechanical fastening members are contacted with each other, the hooks or enlarged heads of the pins will be attached to the loops by hooking engagement. The hooks or the pins comprising the enlarged heads can be arranged regularly, particularly with equal distances from each other, or irregularly, again particularly with equal distances from each other. Preferably, use is made of mechanical fastening devices, of which one member comprises pins having heads arranged at their ends, these headed pins being arranged with a density of about 80 - 800 per CM², preferably 200 - 500 per CM² and more preferably about 400 per CM².

In this context, it should be stressed that the use of the laminate tape of the invention does not depend on the concrete configuration of the used two-part mechanical fastening device. Thus, the laminate tape can be used in each type of a mechanical fastening system.

Therefore, in the Z- or S-shaped folded condition of the laminate tape of the invention, the upper side of the mechanical fastening member fixedly connected to the laminate strip in the second or third portions, is arranged to face the respective other portion, i.e. the third or respective second portion. In this configuration, the upper side of this mechanical fastening member is releasably connected to the adhesive layer of the respective portion of the laminate strip. Thus, this releasable connection of the second and third portions of the laminate strip in the folded condition of the laminate tape is obtained by the first mechanical fastening member provided on the laminate tape, notably in that the lower side of the first mechanical fastening member is fixedly connected - i.e. in a manner precluding unintended release - to the second (or, alternatively, the third) portion of the laminate strip, while the upper side of the mechanical fastening member is releasably connected to the third (or, alternatively, the second) portion of the laminate strip, i.e. to that portion of the laminate strip which is arranged opposite to the portion fixedly connected to the lower side of the mechanical fastening member. Since, on the upper side of the mechanical fastening member, no full-faced connection with the adhesive layer of the portion of the laminate strip opposite this upper side can be generated, the folded laminate strip can be pulled apart, i.e. be unfolded, by using merely a low pull-off force.

It is characteristic of the laminate tape of the invention that the manufacture thereof can be started with a laminate strip which provided with an adhesive layer over the full surface thereof. It is only required that the mechanical fastening member is fixedly bonded in one of the two portions having their adhesive layers facing towards each other in the S- or Z-shaped folded configuration of the laminate strip. No special additional adhesive zones are needed for obtaining the cohesion between these two portions of the laminate strip which due to the mechanical fastening member cannot contact each other directly, so that, in this regard, the manufacturing process will not require additional provisions for applying adhesives only in special zones.

To simplify the handling of the inventive laminate tape to be used as a closure strip, the adhesive force between the upper side of the mechanical fastening member of the laminate strip and that portion of the laminate strip which is arranged opposite this upper side, should be as small as possible. In this regard, it is useful to provide the upper side of the mechanical fastening member of the laminate strip with release properties. This can be performed, e.g. , by applying a releasing coating onto the upper side of the mechanical fastening member of the laminate strip. A suitable material for such a coating is, e.g., a poly(dimethylsiloxane) (PDMS) cross-linked by polycondensation (e.g. the material SylOff 294 from Dow Corning).

By way of alternative to the application of a release coating, it is also possible to shape the structure of the upper side of the mechanical fastening member of the laminate strip in such a manner generating only few contact points with the adhesive layer of the portion of the laminate strip arranged opposite to the upper side in the folded condition of the laminate tape.

According to a further alternative, the upper side of the mechanical fastening member of the laminate strip can have parts thereof - i.e. not its whole surface area - provided with a release coating. The relation between the coated surface and the uncoated surface on the upper side of the mechanical fastening member should be selected according to the desired pull-off force, the materials for the adhesive layer and the release coating and, if desired, the configuration of the upper side of the mechanical fastening member fixedly connected to the laminate strip.

To further facilitate the handling of the inventive laminate strip when moving the laminate strip from the folded condition into the stretched-out condition, that end portion of the laminate strip which is hot connected to said one part of the article to be held together by the laminate tape, can be advantageously with an adhesive-free handling end or fingerlift. This adhesive-free fingerlift can be generated, e.g., by leaving the laminate strip without an adhesive layer in this region. Another possibility resides in the provision of a projecting flap on the respective free edge of the portion, said flap being fixedly connected to the adhesive layer of the respective portion. A particularly suited material for such a fingerlift comprising a separate flap fixedly bonded to the laminate strip is polypropylene. Alternatively, the fingerlift can also be realized by folding the laminate strip on this end.

To keep also the first and second portions of the laminate strip in their mutually overlying condition in the folded state of the laminate strip, it is advantageous to provide an adhesive zone in the region of one of these two portions on the second side of the support layer facing away from the adhesive layer. This adhesive zone provides for a releasable connection between the first and second portions of the laminate strip on the second side of the support layer of the laminate strip. In this manner, in the folded condition of the laminate strip, the respectively adjacent portions are releasably connected to each other while being secured against undesired release.

Suitably, the folded laminate strip is provided as an endless laminate tape in the direction transverse to its lengthwise dimension. The individual laminate strips are produced by cutting off individual strips from the zigzag-shaped folded endless laminate tape, the width of said strips being identical to the width of the desired laminate strips. Suitably, the endless laminate tape is wound into a roll, with the winding direction of the roll extending transversely to the lengthwise direction of the individual laminate strips. i.e. in the lengthwise direction of the endless laminate tape.

### Brief Description of the Drawings

An embodiment of the inventive laminate tape will be described in greater detail hereunder with reference to the Figures; by way of example, the laminate tape will be explained primarily as used for the closure of a disposable diaper.

Fig. 1 is a elevational view onto the outer side of a disposable diaper.

Fig. 2 is a disposable diaper, is a sectional view along the line II-II of Fig. 1, with Fig. 2A showing an enlarged representation of the mechanical fastening member.

Fig. 3 is a plan view of the waist region of the diaper in the closed condition.

Fig. 4 is a view of the folded laminate tape in the rolled condition.

Figs. 5 to 7 are views for illustrating some of the steps of the process for producing the laminate strip.

### Detailed Description

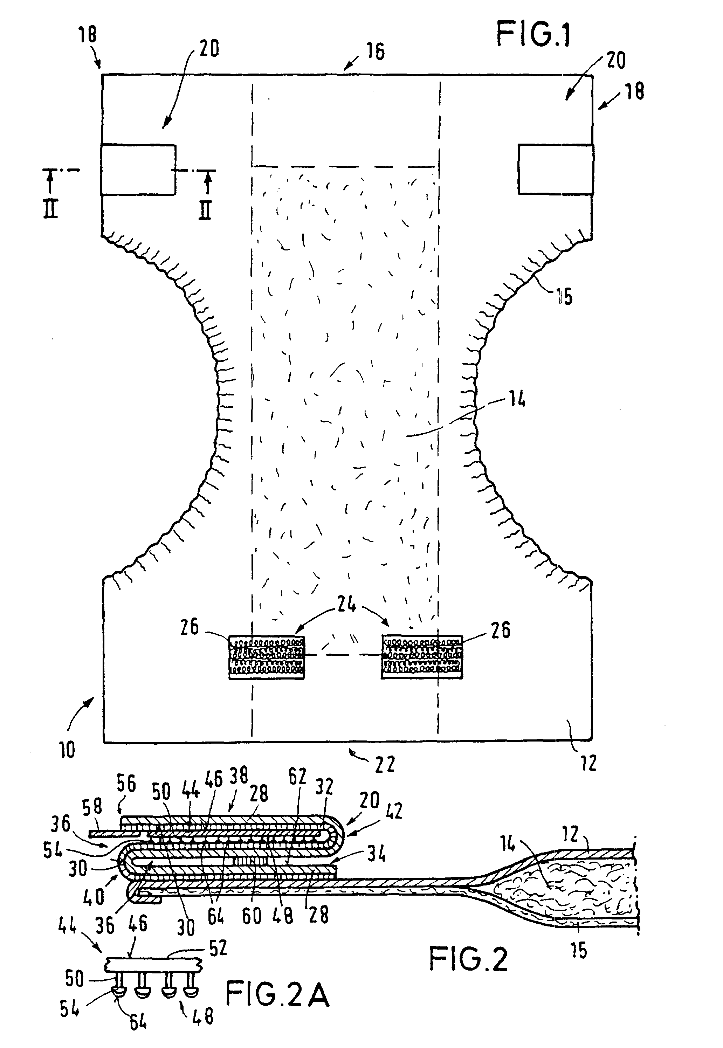

Fig. 1 shows a plan view of the outer side of a disposable diaper 10 for babies. Diaper 10 has its outer side provided with a film 12 impermeable to humidity.

Below said outer film 12, a soft non-woven inlet 14 is arranged in the central region of diaper 10. on the side of said non-woven inlet 14 facing away from outer film 12, one or a plurality of tear resistant non-woven films 15 are arranged, of which the overall cutting shape is equal to the shape of outer film 12 and which, in the edge region projecting beyond non-woven inlet 14, are connected to outer film 12. In the upper edge portion 16 of diaper 10 as viewed in the orientation of Fig. 1, two S- or Z-shaped folded closure band 20 are arranged on the two lateral side edges 18 of edge portion 16. These closure bands 20 are bonded to outer film 12. Arranged in the lower portion 22 of diaper 10 according to Fig. 1 are two central mechanical fastening members 24 which likewise applied from the outside to outer film 12 and are fixedly connected to diaper 10. Said two mechanical fastening members 24 respectively form one part of a two-part mechanical fastening device, the second part or complementary fastening member of the fastening device forming a part of each closure band 20. As schematically shown in Fig. 1, the mechanical fastening members 24 arranged in the lower portion 22 of diaper 10 comprises a plurality of projecting loops 26. In combination with projecting hooks of the fastening members of closure bands 20, these loops 26 form a mechanical fastening device operating in the manner of a hook-and-loop-type fastener. The arrangement of the fastening members of the closure bands 20 will be explained in greater detail hereunder in connection with the description of the design of the closure bands 20.

The design of a closure band 20 will be explained with reference to Fig. 2. The closure band 20 is a laminate strip folded in a Z-shaped configuration, comprising a support layer 28 made of a synthetic material and having one side 30 provided with an adhesive layer 32 over the full surface thereof. Laminate strip 20 is divided into three portions of substantially equal length, notably a first portion 34, a second portion 36 following the first portion 34, and a third portion 38 following the second portion 36. The first and third portions 34 and 38 form the end portions of laminate strip 20 while the second portion 36 is arranged in the middle between these two end portions. In the transition regions 40 and 42 between the portions 34 and 36 and resp. the portions 36 and 38, laminate strip 20 is folded respectively by about 180°, resulting in the Z-folded structure shown in Fig. 2 wherein all of the three portions 34 to 38 are arranged substantially in parallel to each other.

In the folded condition of laminate strip 20, its adhesive layer 32 in the region of the first portion 34 is arranged externally, i.e. on the side of support layer 28 facing away from the second portion 36. As a result, the adhesive layer 32 in the region of the intermediate second portion 36 of adhesive layer 32 is arranged opposite to the laminate strip in the region of the third portion 38 when the laminate strip has been folded into a Z-shape.

As already mentioned above, the laminate strip 20 comprises one of two mechanical fastening members of a two-part mechanical fastening device of which the second fastening member 24 is attached externally on the diaper 10. In the Figures, the first mechanical fastening member attached to laminate strip 20 is designated by reference numeral 44. This first mechanical fastening member 44 has a lower side 46 by which it is fixedly connected to the adhesive layer 32 in the region of the third portion 38 of laminate strip 20. On its upper side 48 facing away from lower side 46, the first mechanical fastening member 44 comprises a plurality of pins 50 which are secured in a substrate layer 52 of mechanical fastening member 44, the free ends of pins 50 facing away from substrate layer 52 have a thickened shape as shown in the Figures at 54. The first mechanical fastening member 44 is produced by a synthetic material using a technique known as such.

In the folded condition of laminate strip 20, the thickened head portions 54 of the first mechanical fastening member 44 abut the adhesive layer 32 of laminate strip 20 in the region of the second portion 36 of laminate strip 20, such that said head portions 54 are releasably attached to adhesive layer 32. Since the contact area of the upper side 48 of the first mechanical fastening member 44 to the adhesive layer 32 is smaller than the contact area of the lower side 46 of the mechanical fastening member 44, the two portions 36 and 38 of laminate strip 20 can relatively easily released from each other while the first mechanical fastening member 44 with its lower side 46 remains fixedly connected to the adhesive layer 32 in the region of the third portion 38 of laminate strip 20.

As illustrated in Fig. 2, laminate strip 20 comprises a flap 58 projecting beyond the free end 56 of the third portion 38 facing away from the second portion 36, said flap 58, like the first mechanical fastening member 44, being fixedly connected to the adhesive layer 32 in the region of the third portion 38 of laminate strip 20. Flap 58 serves as the fingerlift of the laminate strip 20 where the laminate strip 20 is gripped to be moved from the folded condition into the unfolded condition. To secure the two first and second portions 34 and 36 of the laminate strip against undesired unfolding in the folded condition of the laminate strip, a small adhesive strip 60 is arranged between the mutually confronting regions of the support layer of the f first and second portions 34 and 36. By this adhesive strip 60, the second side 62 of support layer 28 facing away from the adhesive layer 32 is connected in the region of the first and second portions 34 and 36. The second side 62 of support layer 28 has its contact areas releasably connected to adhesive strip 60.

Basically, it is provided that the second side 62 has release properties which are obtained by a corresponding coating on this side of support layer 28. (Typically, said coating is provided by a silicone compound or a polyurethane separating agent.) Alternatively, it can be provided that the material for support layer 28 itself has intrinsic release properties and that the side of support layer 28 carrying the adhesive layer 32 is coated with an adhesive agent (primer) on which the material of adhesive layer 32 will then adhere.

As already briefly mentioned above, the adhesive layer 32 in the region of the second portion 36 of laminate strip 20 primarily has the purpose to hold the second and third portions 36 and 38 of laminate strip 20 in their positions according to Fig. 2 by abutment on the upper side 48 of the first mechanical fastening member 44, and to keep said portions from being unfolded unintentionally. While the adhesive force of the adhesive layer 32 in the second portion 36 of laminate strip 20 should thus be relatively low so that the laminate strip 20 can be unfolded already by application of a low force, the material in the adhesive layer in the first and third portions 34 and 38 of laminate strip 20 must have a maximum adhesive force. Notably, according to Fig. 2, the adhesive connection to the outer film 12 of diaper 10 occurs in the first portion 34 of laminate strip 20. In this region, the adhesive force has to be large to keep the laminate strip 20 reliably attached to diaper 10. Also the attachment of the lower side 46 of the first mechanical fastening member 44 to the laminate strip 20 has to be reliable; for this reason, the adhesive layer 32 must guarantee a continuously high adhesive force also in the region of the third portion 38 of laminate strip 20. In order to allow the upper side 48 of the first mechanical fastening member 44 to be released from the adhesive layer 32 without any noteworthy use of force even if an adhesive generating a high adhesive force has been used for the adhesive layer 32, the upper side 48 of the first mechanical fastening member 44, i.e. the thickened head portions of the first mechanical fastening member 44, is provided with a release coating, illustrated in the Figures at 64. Said release coating 64 reduces the adhesion between the upper side 48 of the first mechanical fastening member 44 and the adhesive layer 32 in the region of the intermediate portion 36 of laminate strip 20. Thus, laminate strip 20 can be stretched without considerable application of force by gripping it on the flap 58 forming the fingerlift. In the process, also the first portion 34 or resp. the second portion 36 of laminate strip 20 are easily released from the adhesive strip 60, wherein this effect can be obtained by corresponding characteristics of the surface of support layer 28 and/or suitable selection of the adhesive material for adhesive strip 60.

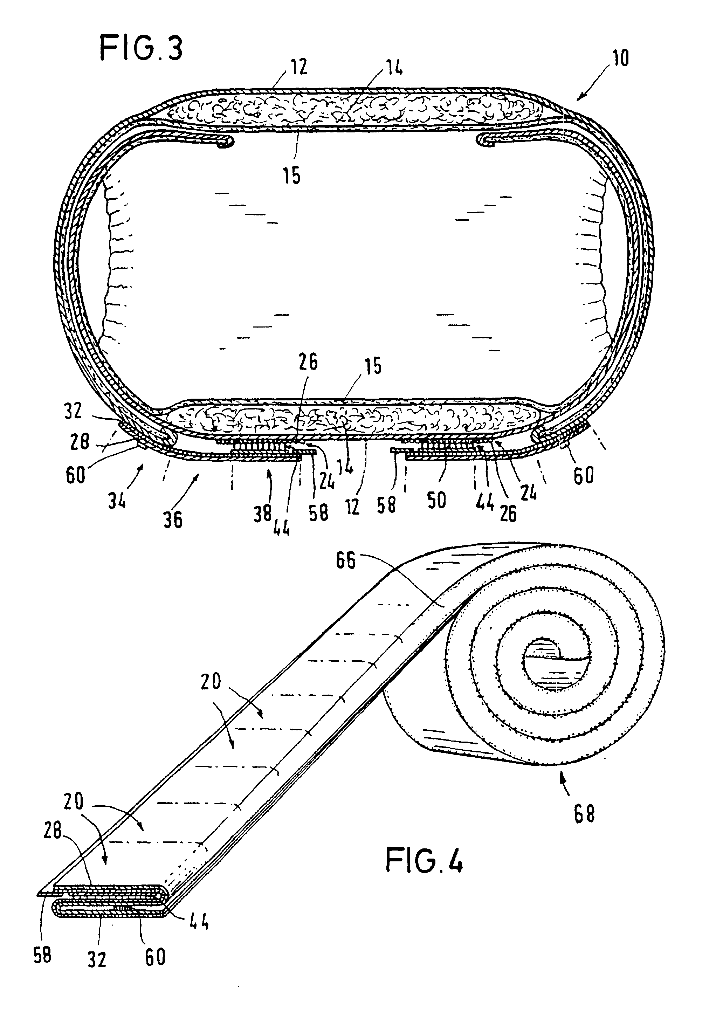

Fig. 3 shows a plan view of the waist region of the closed diaper 10, said waist region being defined by the upper and lower portions 16 and 22 according to Fig. 1. Fig. 3 illustrates the manner in which the hooking elements, formed as the pins 50 of first mechanical fastening member 44 of laminate strip 20 provided with thickened head portions 54, are hooked in the loops 26 of the second mechanical fastening member 24 on the outer side of diaper 10. This manner of a releasable connection of the laminate strips 20 to the diaper 10 allows for a repeated opening and closing of diaper 10 without a decrease of the closing force. Further, this mechanical closure mechanism will advantageously function even if the mechanical fastening members have accidentally been soiled with grease or oil, as happens easily when diapering baby or a toddler. After the diaper 10 has been used, the adhesive layer 32, being exposed in the central portion 36 of the stretched laminate strip 20, can be utilized for holding the folded diaper together.

Fig. 4 shows a zigzag shaped folded laminate tape 66 wound in its folded condition into a roll 68. As evident from Fig. 4, the laminate tape 66 comprises all of the components of the laminate strip 20 of Fig. 2. The individual laminate strips 20 are generated by cutting the laminate tape 66 along its length, i.e. by cutting off individual pieces of laminate tape 66 transversely to its lengthwise direction, as schematically shown in Fig. 4.

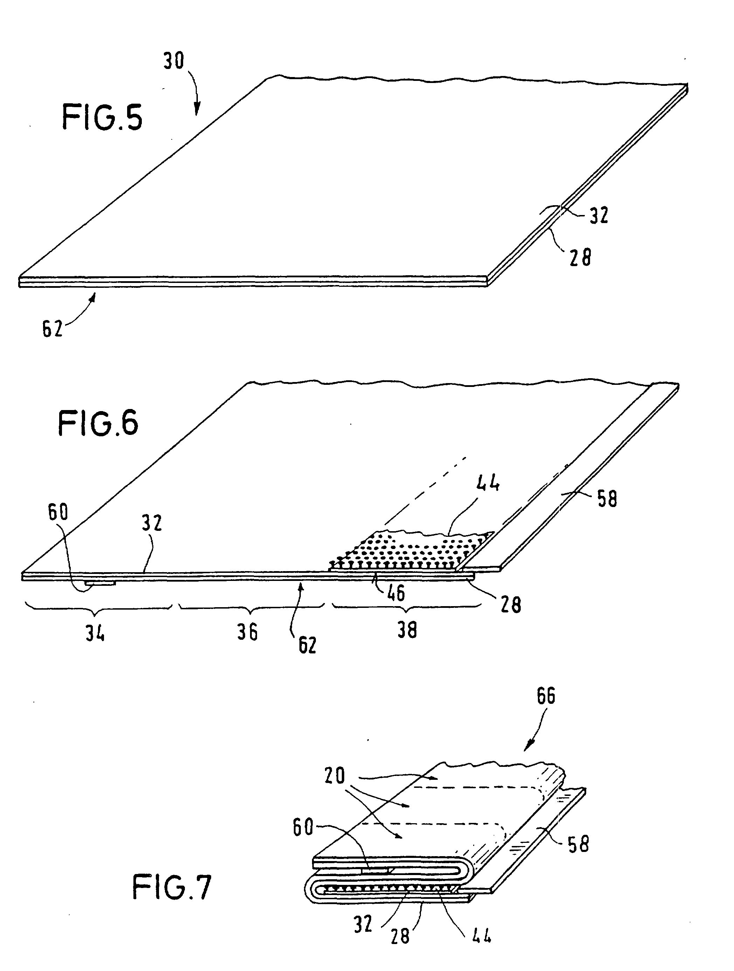

The manufacture of laminate tape 66 according to Fig. 4 is started with a strip-shaped support layer 28 having one side 30 provided with the adhesive layer 32 (Fig. 5). On one of the two lateral strips of the support layer strip 28, there is attached a small strip which is fixedly connected to adhesive layer 32 and laterally projects beyond support layer 28 (Fig. 6). This projecting strip forms the flaps 58 or fingerlift of the later laminate strip 20. Laterally of the projecting strip connected to adhesive layer 32, there is then attached a strip-shaped first mechanical fastening member 44 which by its underside 46 is fixedly connected to adhesive layer 32.

When viewed in the width direction of the stretched-out support layer strip 28, the strip-shaped first mechanical fastening member 44 extends within a lateral third portion 38. Subsequently, in the intermediate third 36 or in the other lateral third 34 of the support layer strip 28, the side 62 of support layer strip 28 facing away from adhesive layer 32 is provided with the releasable adhesive strip 60. Then, this structure is folded into a Z-shaped layered laminate tape 60 (Fig. 7) which will then be wound up to form a roll 68 to be further processed or transported. To produce the diaper 10, small strips 20 are cut off the laminate tape 66 of roll 68; these strips will then be bonded as closure strips onto the outer side of diaper 10 according to Figs. 1 and 2.

### Example

A laminate strip 20 was produced according to the teaching of the invention. This laminate strip 20 had a length dimension of 9.3 cm and a width dimension of 2.5 cm. The support layer 28 of the laminate strip consisted of a polypropylene material having a thickness of 0.11 mm. An adhesive layer 32 having a thickness of 0.035 mm was applied onto one side 30 of this support layer 28. This adhesive layer 32 consisted of a synthetic rubber/adhesive resin material.

The flap 58 connected to adhesive layer 32 had the same width as the laminate strip 20 and a dimension of 0.8 cm in the lengthwise direction of laminate strip 20. This flap 58 had a thickness of 0.11 mm and consisted of a polypropylene material which was provided with a release layer of polydimethylsiloxane on that side which was not connected to the adhesive layer 32. The flap 58 projected 0.1 cm beyond the laminate strip 20. Laterally of flap 58, a first mechanical fastening member 44 was attached, having a dimension of 2.7 cm in the lengthwise direction of laminate strip 20. The first mechanical fastening member 44 had a thickness of 0.40 mm. The first mechanical fastening member 44 was provided with a substrate layer 52 of a thickness of 0.12 mm, with pins 50 having thickened head portions projecting from one side of substrate layer 52. The height of these pins 50 inclusive of the thickened head portions 54 was 0.28 mm. The hooking elements of the first mechanical fastening member 44, consisting of the pins 50 and the thickened head portions 54, were arranged on substrate 52 in a regular orthogonal matrix structure. The number of hooking elements per unit area was 390/CM². The substrate 52, the pins 50 and the thickened head portions 54 consisted of a polypropylene material.

For the release coating 64 of the upper side 48 of the first mechanical fastening member 44 connected to the hooking elements, a PDMS material was selected. In the folded condition and under contact of the coated upper side 58 of the first mechanical fastening member 44 with the adhesive layer 32, the release force was 0.10 N for a pull-off movement at an angle of 900, and 0.15 N for a pull-off movement at a extremely obtuse angle of 1800.

The material selected for the adhesive strip 60 was a synthetic rubber/adhesive resin material. The adhesive force of this material for attachment to the support layer 28 was 2.5 *N*.

Alternative materials for the support layer 28 and the flap 58 are polyethylene and polyester films; for the adhesive layer 32, diverse styrene-isoprene-styrene rubbers; for the release coating 64, silicone acrylates and polyurethanes; and for the adhesive strip 60, ethylvinylacetate, styrene-butadienestyrene rubber and styrene-isoprene-styrene rubber.

## Claims

1. Laminate tape, comprising
- a laminate strip (20) having a lengthwise direction and comprising a support layer (28) and an adhesive layer (32) on a first face (30) of the support layer (28), the laminate strip (20) comprising a first, a second and a third portion (34,36,38) which in this order are arranged successively to each other when viewed in said lengthwise direction, and
- a first mechanical fastening member (44) of a two-part mechanical fastening device, said two-part mechanical fastening device comprising two mechanical fastening members (24,44) which can be connected to each other while generating a retaining force, wherein
- the first mechanical fastening member (44) comprises a lower side (46) and an upper side (48), said upper side (48) being capable of cooperating with the second mechanical fastening member (24) of the two-part mechanical fastening device,
- the first mechanical fastening member (44) has its lower side (46) fixedly connected to the adhesive layer (32) within the second or third portions (36,38) of the laminate strip (20), and the laminate strip (20) is folded in such a manner that said second and said third portions (36,38) of the laminate strip (20) have their adhesive layers (32) arranged opposite each other on the first face (30) of the support layer (28) and that the first mechanical fastening member (44) is arranged between said second and third portions (36,38) of the laminate strip (20), wherein the upper side (48) of the first mechanical fastening member (44) is releasably attached to the adhesive layer (32) of that portion (36;38) of the laminate strip (20) which is arranged opposite to the portion (38;36) of the laminate strip (20) fixedly connected to the lower side (46) of the first mechanical fastening member (44).

2. The laminate tape according to claim 1, **characterized in that** the upper side (48) of the first mechanical fastening member (44) has release properties to facilitate removal of the second or third portions (36,38) of the laminate strip (20) opposite to said upper side (48) from the adhesive layer (32) of the laminate strip (20)

3. The laminate tape according to claim 2, **characterized in that** the upper side (48) of the first mechanical fastening member (44) has a release coating (64) provided thereon.

4. The laminate tape according to any one of claims 1 to 3, **characterized in that** the first mechanical fastening member (44) has its upper side (48) provided with hooking elements (50,54) for hooking engagement with associated loop elements (26) of the second mechanical fastening member (24) of the two-part mechanical fastening device.

5. The laminate tape according to claim 4 and 2 or 3, **characterized in that** said hooking elements (50,54) have free ends at least some of which are provided with a release coating (64).

6. The laminate tape according to claim 4 or 5, **characterized in that** the hooking elements (50, 54) are distributed in a regular manner.

7. The laminate tape according to claim 6, **characterized in that** the hooking elements (50,54) are arranged respectively at equal distances from each other.

8. The laminate tape according to any one of claims 3 to 7, **characterized in that** the hooking elements are formed as pins (50) integrally projecting from a carrier substrate and having their free ends provided with enlarged heads (54).

9. The laminate tape according to any one of claims 1 to 8, **characterized in that** the adhesive layer (32) is continuous in the region of the second or third portions (36,38) of the laminate strip (20) releasably connected to the upper side (48) of the first mechanical fastening member (44).

10. The laminate tape according to any one of claims 1 to 9, **characterized in that** the lower side (46) of the first mechanical fastening member (44) is fixedly connected to the adhesive layer (32) in aid third portion (38) of the laminate strip (20).

11. The laminate tape according to any one of claims 1 to 10, **characterized in that** an end portion (58) of said third portion (38) of the laminate strip (20) has an adhesive-free fingerlift (58) .

12. The laminate tape according to any one of claims 1 to 11, **characterized in that** an adhesive zone (60) for releasably connecting the second side (62) of the support layer (28) in the region of the first and second portions (34,36) of the laminate strip (20), is arranged in the region of said first or said second portions (34,36) of the laminate strip (20) on the second side (62) of the carrier layer (28) facing away from the adhesive layer (32).

13. The laminate tape according to any one of claims 1 to 12, **characterized in that** the second side (62) of the support layer (28) facing away from the adhesive layer (32) has release properties.

14. The laminate tape according to any one of claims 1 to 13, **characterized in that** the adhesive layer (32) extends on the support layer (28) in a continuous manner.

15. The laminate tape according to any one of claims 1 to 14, **characterized in that** the three portions (34,36,38) of the laminate strip (20) have substantially the same dimension in the longitudinal direction of the laminate strip (20).

16. The laminate tape according to any one of claims 1 to 15, **characterized in that** the laminate strip (20) in the longitudinal direction has a considerably smaller dimension than in a direction transverse to the longitudinal direction.

17. The laminate tape according to any one of claims 1 to 16, **characterized in that** the laminate strip (20) is wound to form a roll (68), the longitudinal direction of the laminate strip (20) extending transversely to the winding direction of said roll (68).

## Patentansprüche

1. Laminatband, das folgendes umfaßt:
- einen Laminatstreifen (20) mit einer Längsrichtung, der eine Stützschicht (28) und eine Klebstoffschicht (32) auf einer ersten Fläche (30) der Stützschicht (28) aufweist, wobei der Laminatstreifen (20) einen ersten, einen zweiten und einen dritten Teil (34, 36, 38) umfaßt, die mit Blickrichtung in dieser Längsrichtung in dieser Reihenfolge nacheinander angeordnet sind, und
- ein erstes mechanisches Befestigungsglied (44) einer zweiteiligen mechanischen Befestigungsvorrichtung, die zwei mechanische Befestigungsglieder (24, 44) umfaßt, welche unter Erzeugung einer Haltekraft miteinander verbunden werden können, wobei
- das erste mechanische Befestigungsglied (44) eine Unterseite (46) und eine Oberseite (48) umfaßt, wobei die Oberseite (48) mit dem zweiten mechanischen Befestigungsglied (24) der zweiteiligen mechanischen Befestigungsvorrichtung zusammenwirken kann,
- wobei die Unterseite (46) des ersten mechanischen Befestigungsglieds (44) innerhalb des zweiten oder dritten Teils (36, 38) des Laminatstreifens (20) mit der Klebstoffschicht (32) fest verbunden ist und der Laminatstreifen (20) so gefaltet ist, daß die Klebstoffschichten (32) des zweiten und des dritten Teils (36, 38) des Laminatstreifens (20) auf der ersten Fläche (30) der Stützschicht (28) einander gegenüber angeordnet sind und daß das erste mechanische Befestigungsglied (44) zwischen dem zweiten und dem dritten Teil (36, 38) des Laminatstreifens (20) angeordnet ist, wobei die Oberseite (48) des ersten mechanischen Befestigungsglieds (44) an der Klebstoffschicht (32) des Teils (36; 38) des Laminatstreifens (20) lösbar befestigt ist, der gegenüber dem Teil (38; 36) des Laminatstreifens (20) angeordnet ist, der mit der Unterseite (46) des ersten mechanischen Befestigungsglieds (44) fest verbunden ist.

2. Laminatband nach Anspruch 1, **dadurch gekennzeichnet, daß** die Oberseite (48) des ersten mechanischen Befestigungsglieds (44) Trenneigenschaften aufweist, um ein Entfernen des zweiten oder dritten Teils (36, 38) des Laminatstreifens (20) gegenüber der Oberseite (48) von der Klebstoffschicht (32) des Laminatstreifens (20) zu erleichtern.

3. Laminatband nach Anspruch 2, **dadurch gekennzeichnet, daß** die Oberseite (48) des ersten mechanischen Befestigungsglieds (44) mit einer Trennbeschichtung (64) versehen ist.

4. Laminatband nach einem der Ansprüche 1 bis 3, **dadurch gekennzeichnet, daß** die Oberseite (48) des ersten mechanischen Befestigungsglieds (44) mit Hakenelementen (50, 54) zum Hakeneingriff mit dazugehörigen Schlaufenelementen (26) des zweiten mechanischen Befestigungsglieds (24) der zweiteiligen Befestigungsvorrichtung versehen ist.

5. Laminatband nach Anspruch 4 und 2 oder 3, **dadurch gekennzeichnet, daß** die Hakenelemente (50, 54) freie Enden aufweisen, von denen zumindest einige mit einer Trennbeschichtung (64) versehen sind.

6. Laminatband nach Anspruch 4 oder 5, **dadurch gekennzeichnet, daß** die Hakenelemente (50, 54) gleichmäßig verteilt sind.

7. Laminatband nach Anspruch 6, **dadurch gekennzeichnet, daß** die Hakenelemente (50, 54) jeweils in gleichen Abständen voneinander angeordnet sind.

8. Laminatband nach einem der Ansprüche 3 bis 7, **dadurch gekennzeichnet, daß** die Hakenelemente als integral von einem Träger ragende Stifte (50) ausgebildet sind, deren freie Enden mit vergrößerten Köpfen (54) versehen sind.

9. Laminatband nach einem der Ansprüche 1 bis 8, **dadurch gekennzeichnet, daß** die Klebstoffschicht (32) im Bereich des zweiten oder dritten Teils (36, 38) des Laminatstreifens (20), der mit der Oberseite (48) des ersten mechanischen Befestigungsglieds (44) lösbar verbunden ist, durchgehend ist.

10. Laminatband nach einem der Ansprüche 1 bis 9, **dadurch gekennzeichnet, daß** die Unterseite (46) des ersten mechanischen Befestigungsglieds (44) mit der Klebstoffschicht (32) im Bereich des dritten Teils (38) des Laminatstreifens (20) fest verbunden ist.

11. Laminatband nach einem der Ansprüche 1 bis 10, **dadurch gekennzeichnet, daß** ein Endteil (58) des dritten Teils (38) des Laminatstreifens (20) einen klebstofffreien Fingerlift (58) aufweist.

12. Laminatband nach einem der Ansprüche 1 bis 11, **dadurch gekennzeichnet, daß** eine Klebstoffzone (60) zur lösbaren Verbindung der zweiten Seite (62) der Stützschicht (28) im Bereich des ersten und des zweiten Teils (34, 36) des Laminatstreifens (20) im Bereich des ersten oder zweiten Teils (34, 36) des Laminatstreifens (20) auf der zweiten Seite (62) der Stützschicht (28) von der Klebstoffschicht (32) abgewandt angeordnet ist.

13. Laminatband nach einem der Ansprüche 1 bis 12, **dadurch gekennzeichnet, daß** die zweite Seite (62) der Stützschicht (28), die von der Klebstoffschicht (32) abgewandt ist, Trenneigenschaften aufweist.

14. Laminatband nach einem der Ansprüche 1 bis 13, **dadurch gekennzeichnet, daß** sich die Klebstoffschicht (32) auf der Stützschicht (28) durchgehend erstreckt.

15. Laminatband nach einem der Ansprüche 1 bis 14, **dadurch gekennzeichnet, daß** die drei Teile (34, 36, 38) des Laminatstreifens (20) in Längsrichtung des Laminatstreifens (20) im wesentlichen die gleiche Abmessung aufweisen.

16. Laminatband nach einem der Ansprüche 1 bis 15, **dadurch gekennzeichnet, daß** der Laminatstreifen (20) in Längsrichtung eine wesentlich geringere Abmessung aufweist als in einer quer zur Längsrichtung verlaufenden Richtung.

17. Laminatband nach einem der Ansprüche 1 bis 16, **dadurch gekennzeichnet, daß** der Laminatstreifen (20) zur Bildung einer Rolle (68) aufgewickelt ist, wobei die Längsrichtung des Laminatstreifens (20) quer zur Aufwickelrichtung der Rolle (68) verläuft.

## Revendications

1. Bande stratifiée, comprenant
- un ruban stratifié (20) ayant une direction longitudinale et comprenant une couche de support (28) et une couche adhésive (32) sur une première face (30) de la couche de support (28), le ruban stratifié (20) comprenant une première, deuxième et troisième portions (34, 36, 38) qui, dans cet ordre, sont agencées successivement les unes après les autres en regardant dans ladite direction longitudinale, et
- un premier élément d'attache mécanique (44) d'un dispositif d'attache mécanique en deux parties, ledit dispositif d'attache mécanique en deux parties comprenant deux éléments d'attache mécanique (24, 44) qui peuvent être connectés l'un à l'autre tout en générant une force de retenue,
- le premier élément d'attache mécanique (44) comprenant un côté inférieur (46) et un côté supérieur (48), ledit côté supérieur (48) étant capable de coopérer avec le deuxième élément d'attache mécanique (24) du dispositif d'attache mécanique en deux parties,
- le premier élément d'attache mécanique (44) ayant son côté inférieur (46) connecté de manière fixe à la couche adhésive (32) dans les deuxième ou troisième portions (36, 38) du ruban stratifié (20), et le ruban stratifié (20) étant plié d'une manière telle que lesdites deuxième et troisième portions (36, 38) du ruban stratifié (20) aient leurs couches adhésives (32) agencées en face l'une de l'autre sur la première face (30) de la couche de support (28) et que le premier élément d'attache mécanique (44) soit agencé entre lesdites deuxième et troisième portions (36, 38) du ruban stratifié (20), le côté supérieur (48) du premier élément d'attache mécanique (44) étant attaché de manière détachable à la couche adhésive (32) de la portion (36, 38) du ruban stratifié (20) qui est agencée en face de la portion (38, 36) du ruban stratifié (20) connectée de manière fixe au côté inférieur (46) du premier élément d'attache mécanique (44).

2. Bande stratifiée selon la revendication 1, **caractérisée en ce que** le côté supérieur (48) du premier élément d'attache mécanique (44) a des propriétés antiadhésives pour faciliter le retrait des deuxième ou troisième portions (36, 38) du ruban stratifié (20) en face dudit côté supérieur (48) de la couche adhésive (32) du ruban stratifié (20).

3. Bande stratifiée selon la revendication 2, **caractérisée en ce qu'**un revêtement antiadhésif (64) est prévu sur le côté supérieur (48) du premier élément d'attache mécanique (44).

4. Bande stratifiée selon l'une quelconque des revendications 1 à 3, **caractérisée en ce que** le côté supérieur (48) du premier élément d'attache mécanique (44) est pourvu d'éléments de crochets (50, 54) pour un engagement d'accrochage avec des éléments de boucles associés (26) du deuxième élément d'attache mécanique (24) du dispositif d'attache mécanique en deux parties.

5. Bande stratifiée selon les revendications 4 et 2 ou 3, **caractérisée en ce que** lesdits éléments de crochets (50, 54) ont des extrémités libres dont au moins certaines sont pourvues d'un revêtement antiadhésif (64).

6. Bande stratifiée selon la revendication 4 ou 5, **caractérisée en ce que** les éléments de crochets (50, 54) sont répartis régulièrement.

7. Bande stratifiée selon la revendication 6, **caractérisée en ce que** les éléments de crochets (50, 54) sont agencés respectivement à égale distance l'un de l'autre.

8. Bande stratifiée selon l'une quelconque des revendications 3 à 7, **caractérisée en ce que** les éléments de crochets sont formés en tant qu'ergots (50) faisant saillie intégralement depuis un substrat porteur et dont les extrémités libres sont pourvues de têtes agrandies (54).

9. Bande stratifiée selon l'une quelconque des revendications 1 à 8, **caractérisée en ce que** la couche adhésive (32) est continue dans la région des deuxième ou troisième portions (36, 38) du ruban stratifié (20) connectées de manière détachable au côté supérieur (48) du premier élément d'attache mécanique (44).

10. Bande stratifiée selon l'une quelconque des revendications 1 à 9, **caractérisée en ce que** le côté inférieur (46) du premier élément d'attache mécanique (44) est connecté fixement à la couche adhésive (32) dans ladite troisième portion (38) du ruban stratifié (20).

11. Bande stratifiée selon l'une quelconque des revendications 1 à 10, **caractérisée en ce qu'**une portion d'extrémité (58) de ladite troisième portion (38) du ruban stratifié (20) a une languette de soulèvement (58) exempte d'adhésif.

12. Bande stratifiée selon l'une quelconque des revendications 1 à 11, **caractérisée en ce qu'**une zone adhésive (60) pour connecter de manière détachable le deuxième côté (62) de la couche de support (28) dans la région des première et deuxième portions (34, 36) du ruban stratifié (20), est prévue dans la région de ladite première ou de ladite deuxième portion (34, 36) du ruban stratifié (20) sur le deuxième côté (62) de la couche de support (28) opposé à la couche adhésive (32).

13. Bande stratifiée selon l'une quelconque des revendications 1 à 12, **caractérisée en ce que** le deuxième côté (62) de la couche de support (28) opposé à la couche adhésive (32) a des propriétés antiadhésives.

14. Bande stratifiée selon l'une quelconque des revendications 1 à 13, **caractérisée en ce que** la couche adhésive (32) s'étend sur la couche de support (28) de manière continue.

15. Bande stratifiée selon l'une quelconque des revendications 1 à 14, **caractérisée en ce que** les trois portions (34, 36, 38) du ruban stratifié (20) ont substantiellement la même dimension dans la direction longitudinale du ruban stratifié (20).

16. Bande stratifiée selon l'une quelconque des revendications 1 à 15, **caractérisée en ce que** le ruban stratifié (20) dans la direction longitudinale a une dimension considérablement plus petite que dans une direction transversale à la direction longitudinale.

17. Bande stratifiée selon l'une quelconque des revendications 1 à 16, **caractérisée en ce que** le ruban stratifié (20) est enroulé pour former un rouleau (68), la direction longitudinale du ruban stratifié (20) s'étendant transversalement à la direction d'enroulement dudit rouleau (68).
